# EUROPÄISCHE PATENTSCHRIFT

(11) **EP 1 215 197 B1**
(45) Veröffentlichungstag und Bekanntmachung des Hinweises auf die Patenterteilung: **23.02.2005**
(21) Anmeldenummer: 01128602.8
(22) Anmeldetag: 30.11.2001
(51) Int. Cl.: C07C 209/88, C07C 209/68, C07C 213/02, C07C 217/08, C07B 55/00

(54) **Verfahren zur Racemisierung von optisch aktiven Aminen**
Method for the racemisation of optically active amines
Procédé de racémisation d'amines optiquement actives

(30) Priorität: 15.12.2000 DE 10062729
(43) Veröffentlichungstag der Anmeldung: 19.06.2002
(73) Patentinhaber: BASF AKTIENGESELLSCHAFT, 67056 Ludwigshafen (DE)
(72) Erfinder: Funke, Frank, Dr., 68159 Mannheim (DE); Liang, Shelue, Dr., 67071 Ludwigshafen (DE); Kramer, Andreas, Dr., 67098 Bad Dürkheim (DE); Stürmer, Rainer, Dr., 67127 Rödersheim-Gronau (DE); Höhn, Arthur, Dr., 67281 Kirchheim (DE)

(56) Entgegenhaltungen:
- WO-A-00/29357
- WO-A-00/47546
- WO-A-00/56699
- US-A- 3 923 694

## Beschreibung

Die vorliegende Erfindung betrifft ein Verfahren zur Racemisierung von optisch aktiven Aminen der Formel I 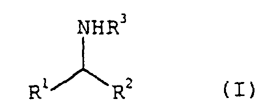 in der R¹ und R² verschieden sind und R¹, R², R³ Alkyl-, Cycloalkyl-, Arylalkyl-, Aryl-, Heteroarylreste und heterocyclische Reste bedeuten und R³ zusätzlich Wasserstoff (H) bedeuten kann, wobei die Reste Substituenten, ausgewählt aus der Gruppe Alkyl, Cycloalkyl, Alkoxy, Aryloxy, Amino, Alkylamino und Dialkylamino, tragen können, durch Umsetzung des optisch aktiven Amins I in Gegenwart von Wasserstoff und einem Hydrier- oder Dehydrierkatalysator bei erhöhter Temperatur.

Optisch aktive Amine der Formel I sind z. B. wertvolle Pharmazeutika und Zwischenprodukte zur Herstellung von Wirkstoffen (vergl. z. B.: DE-A-29 03 589, Seite 2, Zeilen 17 bis 26). Da häufig nur eines der beiden Enantiomeren (bezogen auf das in der Formel I gezeigte asymmetrische C-Atom) wirksam ist, oder wirksamer als das andere Enantiomer ist, werden Verfahren zur Racemisierung des weniger wirksamen Enantiomers, das z. B. bei einer Racematspaltung des entsprechenden racemischen Amins nach bekannten Methoden anfällt, benötigt, weil aus dem racemisierten Amin nach bekannten Methoden (z. B. Racematspaltung) wieder das wirksamere Enantiomer gewonnen werden kann.

Chem. Abstracts 110: 192247v (IN-A-162 213) offenbart ein Verfahren zur Herstellung von racemischem 2-Amino-butanol durch Behandlung von *1*-2-Amino-butanol mit Ammoniak in Gegenwart von Rh/Al₂O₃.

US-A-4,096,186 beschreibt ein Verfahren zur Racemisierung von optisch aktiven Aminoalkoholen, wobei der Aminoalkohol mit Ammoniak und Wasserstoff in Gegenwart eines Hydrierkatalysators, der bevorzugt Cobalt enthält, in Kontakt gebracht wird. Bei der Umsetzung von optisch aktivem 2-Amino-1-butanol wird bei einer Racematausbeute von maximal 97,6 % nur ein Racemisierungsgrad von 63 % erreicht. Bei einem Racemisierungsgrad von 99 % wird dagegen nur eine Racematausbeute von 75,1 % erreicht.

US-A-4,990,666 offenbart ein Verfahren zur Racemisierung von optisch aktiven Aminoalkoholen, wobei der Aminoalkohol in Gegenwart von Wasserstoff mit Raney-Cobalt in Kontakt gebracht wird. Es wird gelehrt, dass hohe Temperaturen, z. B. über 160 °C, die Racematausbeute herabsetzen.

Derwent Abstract Nr. 94-197043/24 (Chem. Abstracts 121: 179093z; JP-A-06 135 906) beschreibt ein Verfahren zur Racemisierung von optisch aktiven vicinalen primären Diaminen in Gegenwart von Wasserstoff und einem Hydrierkatalysator, wie z. B. Raney-Nickel und Raney-Cobalt.

Patent Abstracts of Japan, Vol. 12, No. 467 (C-550), (JP-A-63 185 943), beschreibt die Racemisierung von optisch aktivem 1-Methyl-3-phenylpropylamin in Gegenwart von Raney-Nickel oder Raney-Cobalt und Wasserstoff bei Temperaturen von 50-200 °C.

US-A-3,954,870 offenbart eine Methode zur Racemisierung von bestimmten optisch aktiven alpha,beta-Diphenylethylaminen durch Erhitzen in Gegenwart von trockenem Raney-Nickel.

DE-A-28 51 039 beschreibt ein Verfahren zur Herstellung racemischer Gemische aus optisch aktiven 1-Arylaminen, wobei man die optisch aktiven 1-Arylamine in Gegenwart eines Hydrierkatalysators, insbesondere Raney-Cobalt, mit Wasserstoff behandelt.

DE-A-29 03 589 beschreibt ein Verfahren zur Herstellung racemischer Gemische aus optisch aktiven Aminen, indem man die optisch aktiven Amine mit Wasserstoff in Gegenwart eines Hydrierkatalysators, insbesondere Raney-Cobalt oder Raney-Nickel, bei erhöhter Temperatur behandelt. Die Umsetzung von optisch aktivem 2-Amino-1-phenylpropan an einem Raney-Cobalt-Katalysator bei einer Reaktionsdauer von 12 Stunden führt bei einem Racemisierungsgrad von maximal 98 % nur zu einer Racematausbeute von 91,1 %.

WO 00/29357 betrifft ein Verfahren zur Racemisierung von optisch aktiven Aminen durch Umsetzung des optisch aktiven Amins in Gegenwart von Wasserstoff und einem Hydrier- oder Dehydrierkatalysator bei erhöhter Temperatur, indem man die Umsetzung in der Gasphase durchführt.

WO 00/47546 offenbart ein Verfahren zur Racemisierung von optisch aktiven Aminen durch Umsetzung des optisch aktiven Amins in Gegenwart von Wasserstoff und einem Hydrier- oder Dehydrierkatalysator bei erhöhter Temperatur, indem man die Umsetzung in flüssiger Phase durchführt und der Katalysator die katalytisch aktiven Bestandteile Kupfer, Silber, Eisen, Cobalt, Nickel, Ruthenium, Rhodium, Palladium, Osmium, Iridium und/oder Platin und ein Trägermaterial, ausgewählt aus der Gruppe Aluminiumoxid, Zirkoniumdioxid, Titandioxid, Kohlenstoff und/oder sauerstoffhaltige Verbindungen des Siliziums, enthält.

WO 00/47545 (Äquivalent: US-A-6,049,007) beschreibt ein Verfahren zur Herstellung von racemischen Aminen, indem man gleichzeitig in situ ein entsprechendes optisch aktives Amin und einen entsprechenden sekundären Alkohol und/oder ein entsprechendes unsymmetrisches Keton und ein bestimmtes Amin in Gegenwart von Wasserstoff und einem Hydrier- oder Dehydrierkatalysator bei erhöhter Temperatur umsetzt.

US-A-6,060,624 betrifft ein Verfahren zur Racemisierung von bestimmten optisch aktiven primären β-Alkoxy-alkylaminen durch Umsetzung an einem Nickel- oder Cobalt-Katalysator in Gegenwart von Wasserstoff und Ammoniak.

Nachteilig an einigen Verfahren des Stands der Technik ist auch, dass teure Edelmetall-Katalysatoren eingesetzt werden.

Der vorliegenden Erfindung lag die Aufgabe zugrunde, ein verbessertes wirtschaftliches Verfahren zur Racemisierung von optisch aktiven Aminen aufzufinden, bei dem das Verfahrensprodukt mit hohem Racemisierungsgrad bei gleichzeitig hoher Racemisierungsausbeute (Racematausbeute) und hoher Raum-Zeit-Ausbeute erhalten wird.

Demgemäß wurde ein Verfahren zur Racemisierung von optisch aktiven Aminen der Formel I 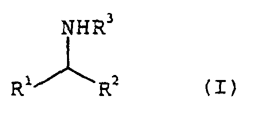 in der R¹ und R² verschieden sind und R¹, R², R³ Alkyl-, Cycloalkyl-, Arylalkyl-, Aryl-, Heteroarylreste und heterocyclische Reste bedeuten und R³ zusätzlich Wasserstoff (H) bedeuten kann, wobei die Reste Substituenten, ausgewählt aus der Gruppe Alkyl, Cycloalkyl, Alkoxy, Aryloxy, Amino, Alkylamino und Dialkylamino, tragen können, durch Umsetzung des optisch aktiven Amins I in Gegenwart von Wasserstoff und einem Hydrier- oder Dehydrierkatalysator bei erhöhter Temperatur gefunden, welches dadurch gekennzeichnet ist, daß der Katalysator die Aktivkomponenten Kupfer und Zinkoxid und ein Trägermaterial enthält.

In einer besonderen Ausführungsform enthält der Katalysator ein oxidisches Trägermaterial, ausgewählt aus der Gruppe Aluminiumoxid, Siliziumdioxid, Titandioxid, Zirkoniumdioxid, Magnesiumoxid, Eisenoxid und Cerdioxid, oder als Trägermaterial Kohlenstoff oder Gemische hiervon.

Aluminiumoxid ist als Trägermaterial bevorzugt.

Das erfindungsgemäße Verfahren lässt sich in der Flüssigphase oder bevorzugt in der Gasphase, diskontinuierlich oder bevorzugt kontinuierlich wie folgt durchführen, wobei der Katalysator bevorzugt als Festbett im Reaktor angeordnet ist.

Das erfindungsgemäße Verfahren kann in Abwesenheit oder bevorzugt in Gegenwart des Amins der Formel R³NH₂, bei dem der Rest R³ dem Rest R³ des optisch aktiven Amins I entspricht, (z. B. des Amins Ammoniak im Fall der Racemisierung von optisch aktiven Aminen I, bei denen R³ = H ist) durchgeführt werden.

Wird in Gegenwart des Amins R³NH₂ gearbeitet, so beträgt im allgemeinen das Molverhältnis von R³NH₂ zu Amin I 1 : 1 bis 50 : 1, bevorzugt 1,5 : 1 bis 30 : 1, besonders bevorzugt 2 : 1 bis 20 : 1, ganz besonders bevorzugt 2 : 1 bis 10 : 1. Der R³NH₂-Überschuß bezogen auf das Amin I kann auch größer als 50 : 1 sein.

Der Wasserstoff wird der Reaktion im allgemeinen in einer Menge von 5 bis 400 l, bevorzugt in einer Menge von 10 bis 200 l, pro Mol Aminkomponente I zugeführt, wobei die Literangaben jeweils auf Normalbedingungen umgerechnet sind (S.T.P.).

Bei der Durchführung des erfindungsgemäßen Verfahrens in der Gasphase wird das optisch aktive Amin I in einem Reaktor, z. B. einem von außen beheiztem Rohrreaktor, in einem zur Verdampfung ausreichend groß gewählten Gasstrom enthaltend Wasserstoff und vorteilhaft das Amin R³NH₂, bevorzugt bestehend aus Wasserstoff und dem Amin R³NH₂, bei Drücken von im allgemeinen 0,1 bis 30 MPa, insbesondere 0,1 bis 10 MPa, bevorzugt 0,1 bis 5 MPa, besonders bevorzugt 0,1 bis 3 MPa, gasförmig kontinuierlich über den Katalysator geleitet.

Es ist dabei sowohl eine Anströmung des Katalysatorfestbetts von oben als auch von unten möglich. Den erforderlichen Gasstrom erhält man bevorzugt durch eine Kreisgasfahrweise, wobei beispielsweise bei einem Katalysatorschüttvolumen von 1 l eine Kreisgasmenge von ca. 5 bis 10 m³/h (Volumen auf Normalbedingungen umgerechnet) und eine Abgasmenge von ca. 250 bis 350 l/h gefahren wird. Die Katalysatorbelastung liegt im allgemeinen im Bereich von 0,1 bis 2, bevorzugt 0,1 bis 1, besonders bevorzugt 0,3 bis 0,8, kg Amin I pro Liter Katalysator (Schüttvolumen) und Stunde.

Die Racemisierung des optisch aktiven Amins I in der Gasphase kann in Gegenwart eines inerten und unter den gewählten Reaktionsbedingungen gasförmigen Verdünnungsmittels, wie Stickstoff und/oder Argon, erfolgen.

Bei der Durchführung des erfindungsgemäßen Verfahrens in der Flüssigphase wird das optisch aktive Amin I in Gegenwart von Wasserstoff und vorteilhaft dem Amin R³NH₂, bei Drücken von 0,1 bis 30 MPa, bevorzugt 5 bis 25 MPa, besonders bevorzugt 10 bis 25 MPa, flüssig über den Katalysator geleitet, der sich üblicherweise in einem bevorzugt von außen beheizten Festbettreaktor, z. B. Rohrreaktor, befindet.

Bei der Durchführung in einem Rohrreaktor ist dabei sowohl eine Anströmung des Katalysatorfestbetts von oben (z. B. Rieselfahrweise) als auch von unten (Sumpffahrweise) möglich. Eine Kreisgasfahrweise ist vorteilhaft, wobei beispielsweise bei einem Katalysatorschüttvolumen von 1 l eine Kreisgasmenge von ca. 0,01 bis 1 m³/h (Volumen auf Normalbedingungen umgerechnet) und eine Abgasmenge von ca. 10 bis 300 l/h gefahren wird.

Die Katalysatorbelastung liegt im allgemeinen im Bereich von 0,05 bis 2, bevorzugt 0,1 bis 1, besonders bevorzugt 0,2 bis 0,6, kg Amin I pro Liter Katalysator (Schüttvolumen) und Stunde.

Die für die Racemisierung in der Flüssigphase und in der Gasphase gewählten Temperaturen liegen im Bereich von 100 bis 300 °C, bevorzugt 150 bis 270 °C, besonders bevorzugt 160 bis 250 °C, ganz besonders bevorzugt 170 bis 240 °C, insbesondere bei 180 bis 230 °C.

Die Racemisierung des optisch aktiven Amins I in der Flüssigphase kann in Gegenwart eines inerten und unter den gewählten Reaktionsbedingungen flüssigen Verdünnungs- oder Lösungsmittels, wie Tetrahydrofuran, Dioxan, N-Methylpyrrolidon und/oder Ethylenglykoldimethylether, erfolgen.

Sowohl bei der Durchführung des Verfahrens in der Gasphase als auch in der Flüssigphase ist die Anwendung höherer Temperaturen, höherer Gesamtdrücke sowie höherer Katalysatorbelastungen als oben angegeben möglich.

Der Druck im Reaktionsgefäß, welcher sich im wesentlichen aus der Summe der Partialdrücke von optisch aktivem Amin I, gegebenenfalls dem Amin R³NH₂, des gebildeten racemisierten Amins I und des gegebenenfalls vorhandenen Verdünnungs- oder Lösungsmittels bei der jeweils angewendeten Temperatur ergibt, wird zweckmäßigerweise durch Aufpressen von Wasserstoff auf den gewünschten Reaktionsdruck erhöht.

In einer besonderen Ausgestaltung wird das Verfahren zur Racemisierung von optisch aktiven Aminen der Formel I so ausgeführt, dass man die Umsetzung in Gegenwart des Amins der Formel R³NH₂, bei dem der Rest R³ dem Rest R³ des optisch aktiven Amins I entspricht, durchführt und gleichzeitig in situ den sekundären Alkohol der Formel II und/oder das unsymmetrische Keton der Formel III 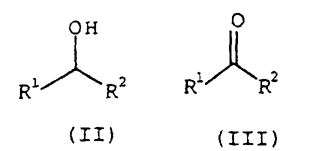 bei denen die Reste R¹ und R² den Resten R¹ und R² des Amins I entsprechen, zum racemischen Amin I umsetzt.

Diese besondere Verfahrensausgestaltung lässt sich durch folgendes Schema illustrieren:

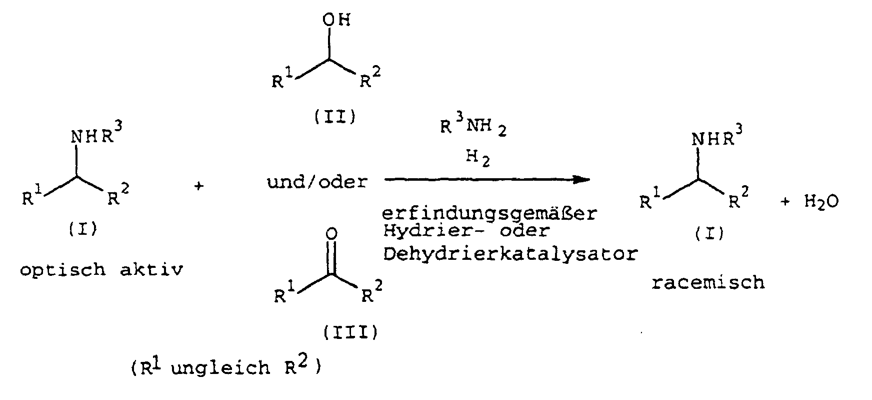

Im allgemeinen beträgt das Molverhältnis von R³NH₂ zur Summe aus optisch aktivem Amin I und Alkohol II und/oder Keton III 1 : 1 bis 50 : 1, bevorzugt 1,5 . 1 bis 30 : 1, besonders bevorzugt 2 : 1 bis 20 : 1, ganz besonders bevorzugt 2 : 1 bis 10 : 1. Der molare R³NH₂-Überschuss bezogen auf die Summe aus optisch aktivem Amin I und Alkohol II und/oder Keton III kann auch größer als 50 : 1 sein.

Das Molverhältnis von optisch aktivem Amin I zu Alkohol II und/oder Keton III ist unkritisch und in weiten Bereichen variierbar und beträgt im allgemeinen 1 : 100 bis 100 : 1, bevorzugt 1 : 50 bis 50 : 1, beispielsweise 1 : 1.

Der Wasserstoff wird der Reaktion im allgemeinen in einer Menge von 5 bis 400 l, bevorzugt in einer Menge von 10 bis 200 l, pro molare Summe aus optisch aktivem Amin I und Alkohol II und/oder Keton III zugeführt, wobei die Literangaben jeweils auf Normalbedingungen umgerechnet sind (S.T.P.).

Die Durchführung dieser besonderen Verfahrensausgestaltung in der Flüssigphase oder bevorzugt in der Gasphase erfolgt sonst wie bereits oben beschrieben, wobei der sekundäre Alkohol der Formel II und/oder das unsymmetrische Keton der Formel III entsprechend mit eingesetzt werden. Die oben angegebenen Katalysatorbelastungen in kg pro Liter Katalysator und Stunde beziehen sich in diesem Fall auf das Edukt-Gemisch [Amin + (Alkohol und/oder Keton)).

Aus dem Reaktionsaustrag werden, nachdem dieser zweckmäßigerweise entspannt worden ist, der Wasserstoff, das eventuell verwendete Amin der Formel R³NH₂ und das gegebenenfalls verwendete Verdünnungs- oder Lösungsmittel entfernt (z. B. destillativ), wobei diese zurückgeführt werden können, und das erhaltene abgekühlte Reaktionsrohprodukt, das im wesentlichen racemisches Amin I enthält, durch eine fraktionierende Rektifikation, bei Normaldruck oder bei vermindertem Druck, gereinigt.

Im Fall der oben beschriebenen besonderen Verfahrensausgestaltung, bei der gleichzeitig *in situ* der sekundäre Alkohol der Formel II und/oder das unsymmetrische Keton der Formel III mit umgesetzt wurde, enthält das Reaktionsrohprodukt zusätzlich Wasser. Das Wasser wird vorzugsweise vor der Durchführung der Rektifikation durch Behandlung mit ca. 50 %iger wässriger Natronlauge entfernt.

Beispielsweise kann gemäß dem erfindungsgemäßen Verfahren die Herstellung von racemischem 1-Methoxy-2-aminopropan ((R,S)-MOIPA) (R¹ = -CH₃, R² = -CH₂OCH₃, R³ = H) erfolgen.

Gemäß der oben beschriebenen besonderen Verfahrensausgestaltung kann gemäß dem erfindungsgemäßen Verfahren beispielsweise die Herstellung von racemischem MOIPA durch gleichzeitige in situ-Umsetzung von optisch aktivem 1-Methoxy-2-aminopropan, 1-Methoxy-2-propanol und Ammoniak erfolgen.

Die Aufarbeitung des gemäß dieses Beispiels erhaltenen Verfahrensrohprodukts, das im wesentlichen (R,S)-MOIPA und Wasser enthält, kann z. B. durch Versetzen des Austrags mit Natronlauge, Abtrennung der wässrigen Phase und Destillation der (R,S)-MOIPAenthaltenden Phase, gemäß EP-A-881 211, erfolgen.

Der zusätzliche Vorteil des erfindungsgemäßen Verfahrens gemäß der oben beschriebenen besonderen Verfahrensausgestaltung liegt u. a. in seiner besonderen Wirtschaftlichkeit, da keine getrennten Anlagen zur Herstellung der racemischen Amine I durch (a) Aminierung von sekundären Alkoholen II oder unsymmetrischen Ketonen III mit Aminen der Formel R³NH₂ und (b) Racemisierung des entsprechenden optisch aktiven Amins I aufgebaut werden müssen, sondern die Verfahren (a) und (b) gleichzeitig in situ durchgeführt werden können. (Vergleiche hierzu auch die Ausführungen auf Seite 1 der Beschreibung, 2. Absatz).

Überraschenderweise werden durch die Zusammenfassung der beiden völlig unterschiedlichen o. g. Verfahrensschritte (a) und (b) in einer einzigen Verfahrensstufe die Ausbeuten und Selektivitäten der einzelnen Verfahrensschritte praktisch nicht beeinflusst. D. h. die verstärkte Bildung von Nebenprodukten, wie zum Beispiel symmetrischen Aminen der Formel 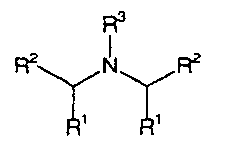 wird praktisch nicht beobachtet.

Erfindungsgemäß wurde erkannt, dass die Verwendung von Nickeloder Cobalt-Katalysatoren in der oben beschriebenen besonderen Verfahrensausgestaltung kritisch sein kann, da diese Katalysatoren die zu aminierenden Alkohole II oder Ketone III leicht in Methan oder Ethan (als Spaltprodukte) umwandeln können (;Spaltungsreaktion'). Die Bildung dieser Spaltprodukte verringert nicht nur die Ausbeuten an racemischem Amin I, sondern ruft auch auch - bei unkontrollierter, verstärkter Spaltung vor allem im adiabatisch betriebenen Reaktor - eine gefährliche, exotherme Durchgehrektion hervor, die zu Temperaturen von über 500 °C im Reaktor führen kann. Erfindungsgemäß zeigen die gefundenen Katalysatoren zum einen selbst bei hohen Temperaturen eine geringe Tendenz zur Spaltungsreaktion (wie oben beschrieben) bei der Aminierung der Alkohole II oder Ketone III und gleichzeitig die oben beschriebenen Vorteile des erfindungsgemäßen Racemisierungsverfahrens (z. B. hoher Racemisierungsgrad bei gleichzeitig hoher Ausbeute an racemischem Amin I).

Die im erfindungsgemäßen Verfahren verwendeten Katalysatoren enthalten die Aktivkomponenten Kupfer und Zinkoxid und ein Trägermaterial.

Die Katalysatoren können sowohl in Pulverform in Suspension als auch vorzugsweise in Form von Voll-, Tränk-, Schalen- oder Fällkatalysatoren im Festbett eingesetzt werden.

Als Trägermaterial werden Oxide der Elemente Aluminium, Silizium, Zirkonium, Titan, Magnesium, Eisen, Cer oder Kohlenstoff oder Gemische dieser Trägermaterialien eingesetzt.

Beispiele für solche Oxide sind Al₂O₃, SiO₂, ZrO₂, TiO₂, MgO, Fe₂O₃ (z. B. Magnetit), CeO₂. Kohlenstoff kann in Form von Aktivkohle als Trägermaterial eingesetzt werden.

Aluminiumoxid (Al₂O₃) wird beispielsweise in Form von α-, β-, γ-oder θ-Al₂O₃ oder D10-10 von BASF eingesetzt.

Besonders bevorzugt wird als Trägermaterial ein Gemisch aus Aluminiumoxid und Zinkoxid eingesetzt.

In einer ganz besonderen Ausführungsform enthält der Katalysator zusätzlich Zink-Aluminium-Spinell. Siehe hierzu z. B. US-A-3,923,694, wo solch ein Katalysator offenbart ist.

Hinsichtlich der Herstellung des Trägermaterials existieren keine besonderen Beschränkungen.

Im allgemeinen kann das oxidische Trägermaterial z. B. durch Fällung einer Zinknitrat und entsprechend Metallsalz, z. B. Aluminiumnitrat, enthaltenden wässrigen Lösung mit Soda und anschließende Filtration, Trocknung und gegebenenfalls Calcinierung des so erhaltenen Niederschlages hergestellt werden.

Der in dem erfindungsgemäßen Verfahren verwendete Katalysator zeichnet sich dadurch aus, dass die Aktivkomponenten Kupfer und Zinkoxid auf das obengenannte Trägermaterial aufgebracht wird, wobei bezüglich der Aufbringungsmethode keinerlei Beschränkungen existieren.

Insbesondere können eine Kupfersalzlösung und eine Zinksalzlösung oder eine Lösung enthaltend Kupfer- und Zinksalz in einer oder mehreren Tränkstufen auf den vorgefertigten Träger in Form von Pulver, Kugeln, Strängen oder Tabletten aufgebracht werden. Der Träger wird im Anschluss an die Tränkung getrocknet und gegebenenfalls calciniert.

Solche Tränkverfahren sind z. B. in EP-A-599 180, EP-A-673 918 oder A. B. Stiles, Catalyst Manufacture - Laboratory and Commercial Preparations, Marcel Dekker, Seiten 89 bis 91, New York (1983), beschrieben.

Auch können eine Kupfersalzlösung und eine Zinksalzlösung oder eine Lösung, enthaltend Kupfer- und Zinksalz, durch Fällung auf den vorgefertigten Träger, der in einer besonders bevorzugten Ausführungsform als Pulver in einer wässrigen Suspension vorliegt, aufgebracht werden. Die Fällung erfolgt nach den im Stand der Technik bekannten Verfahren.

Die aus dem Fällungsprozess erhaltenen Niederschläge werden in üblicher Weise filtriert und vorzugsweise alkalifrei gewaschen - wie dies beispielsweise in der DE-A-198 09 418 beschrieben ist -, bei Temperaturen von 50 bis 150 °C, vorzugsweise bei ca. 120 °C, getrocknet und im Anschluss gegebenenfalls, vorzugsweise ca. 2 Stunden, bei im allgemeinen 200 bis 600 °C, insbesondere bei 300 bis 500 °C, calciniert.

Als Ausgangssubstanzen für die Katalysatorherstellung können prinzipiell alle in den bei der Aufbringung verwendeten Lösungsmitteln löslichen Cu(I)- und/oder Cu(II)-Salze, wie beispielsweise Nitrate, Carbonate, Acetate, Oxalate oder Ammonium-Komplexe, und analoge Zn(II)-Salze verwendet werden. Besonders bevorzugt wird Kupfernitrat und Zinknitrat eingesetzt.

Für das erfindungsgemäße Verfahren wird das oben beschriebene getrocknete und bevorzugt calcinierte Katalysator-Pulver bevorzugt zu Tabletten, Ringen, Ringtabletten, Extrudaten, Wabenkörpern oder ähnlichen Formkörpern verarbeitet. Hierfür sind sämtliche aus dem Stand der Technik geeigneten Verfahren anwendbar.

Bevorzugte Katalysatoren enthalten nach der Trocknung und vor der Aktivierung mit einem Reduktionsmittel (zum Reduktionsmittel siehe unten)
20 bis 90 Gew.-%, besonders 40 bis 85 Gew.-%, ganz besonders 60 bis 80 Gew.-%, sauerstoffhaltige Verbindungen des Kupfers, berechnet als CuO,
9 bis 60 Gew.-%, besonders 13 bis 40 Gew.-%, ganz besonders 17 bis 30 Gew.-% sauerstoffhaltige Verbindungen des Zinks, berechnet als ZnO, und
in Summe 1 bis 60 Gew.-%, besonders 2 bis 35 Gew.-%, ganz besonders 3 bis 10 Gew.-%, der oben genannten oxidischen Trägermaterialien, jeweils berechnet als Al₂O₃, SiO₂, ZrO₂, TiO₂, MgO, Fe₂O₃ bzw. CeO₂, insbesondere Aluminiumoxid (berechnet als Al₂O₃), jeweils bezogen auf das Gesamtgewicht aller oxidischen Bestandteile des Katalysators, wobei Katalysatorverformungshilfmittel (Zemente) nicht zu den oxidischen Bestandteilen gerechnet werden.

Besonders bevorzugte Katalysatoren enthalten nach der Trocknung und vor der Aktivierung mit einem Reduktionsmittel die Bestandteile sauerstoffhaltige Verbindungen des Kupfers, sauerstoffhaltige Verbindungen des Zinks und oben genanntes oxidisches Trägermaterial, insbesondere Aluminiumoxid, zusammen in einer Menge von mindestens 80 Gew.-%, insbesondere mindestens 90 Gew.-%, bezogen auf das Gesamtgewicht aller oxidischen Bestandteile des Katalysators, wobei Katalysatorverformungshilfmittel (Zemente) nicht zu den oxidischen Bestandteilen gerechnet werden.

In besonderen Katalysator-Ausführungsformen enthält der Katalysator, nach der Trocknung und vor der Aktivierung mit einem Reduktionsmittel, in einem Anteil von 0,01 bis 10 Gew.-%, bevorzugt 0,01 bis 5 Gew.-%, beispielsweise 1 bis 10 Gew.-% und bevorzugt 1 bis 5 Gew.-%, jeweils bezogen auf das Gesamtgewicht aller oxidischen Bestandteile des Katalysators (wobei Katalysatorverformungshilfmittel (Zemente) nicht zu den oxidischen Bestandteilen gerechnet werden), mindestens eine weitere Komponente, die ausgewählt wird aus der Gruppe bestehend aus Oxiden der Elemente Re, Fe, Ru, Co, Rh, Ir, Ni, Pd und Pt.

Diese Komponenten können ebenfalls über die bekannten Fällungsoder Tränkungsmethoden in das Katalysatormaterial eingebracht werden.

In dieser besonderen Katalysator-Ausführungsform enthält der Katalysator nach der Trocknung und vor der Aktivierung mit einem Reduktionsmittel 20 bis 89 Gew.-%, besonders 40 bis 84 Gew.-%, ganz besonders 60 bis 79 Gew.-%, sauerstoffhaltige Verbindungen des Kupfers, berechnet als CuO, bezogen auf das Gesamtgewicht aller oxidischen Bestandteile des Katalysators (wobei Katalysatorverformungshilfmittel (Zemente) nicht zu den oxidischen Bestandteilen gerechnet werden).

Bei der Herstellung von Formkörpern .der im erfindungsgemäßen Verfahren verwendeten Katalysatoren können zur besseren Katalysator-Performance (höhere Aktivität, Selektivität, mechanische und chemische Stabilität) pulverförmiges Kupfer oder pulverförmiges Katalysatorverformungshilfmittel (Zement) oder ein Gemisch davon als Additiv vor der Formgebung dem Katalysatormaterial zugesetzt werden.

Im allgemeinen kann hierbei dem Katalysatormaterial pulverförmiges Kupfer oder pulverförmiger Zement oder ein Gemisch davon im Bereich von 1 bis 40 Gew.-%, bevorzugt im Bereich von 2 bis 20 Gew.-% und besonders bevorzugt im Bereich von 5 bis 10 Gew.-%, jeweils bezogen auf das Gesamtgewicht aller oxidischen Bestandteile des Katalysatormaterials, zugesetzt werden.

Beispiele für Katalysatorverformungshilfmittel (Zemente) sind Stearinsäure und Graphit.

In einer bevorzugten Ausführungsform werden Kupferpulver und Zementpulver mit einer Korngrößenverteilung verwendet, bei der mindestens 45 %, bevorzugt mindestens 70 %, besonders bevorzugt mindestens 90 % (Angaben in Gew.-%) der Kupfer- oder Zementpartikel Korngrößen im Bereich 10 bis 100 mm besitzen. Die Oberfläche des verwendeten Kupferpulvers oder Zementpulvers, bestimmt nach der BET-Methode, liegt im allgemeinen im Bereich von 0,01 bis 20 m²/g, bevorzugt im Bereich von 0,05 bis 10 m²/g, besonders bevorzugt im Bereich von 0,1 bis 0,5 m²/g.

In einer weiteren bevorzugten Ausführungsform werden bei der Herstellung von Formkörpern der im erfindungsgemäßen Verfahren verwendeten Katalysatoren zur besseren Katalysator-Performance (s.o.) zusätzlich zu dem Kupferpulver oder dem Zementpulver oder dem Gemisch davon Graphit als Verformungshilfsmittel dem Katalysatormaterial zugesetzt. Vorzugsweise wird dabei soviel Graphit zugegeben, dass die Verformung zu einem Formkörper besser und leichter durchgeführt werden kann. Beispielsweise werden 0,5 bis 5 Gew.-% Graphit, bezogen auf das Gesamtgewicht aller oxidischen Bestandteile des Katalysatormaterials, zugegeben. Dabei ist es gleichgültig, ob Graphit dem Katalysatormaterial vor oder nach oder gleichzeitig mit dem Kupferpulver oder dem Zementpulver oder dem Gemisch davon zugesetzt wird.

Nach Zugabe des Kupferpulvers oder des Zementpulvers oder des Gemischs davon und gegebenenfalls des Graphits zu dem Katalysatormaterial wird der im Anschluss an die Verformung erhaltene Katalysatorformkörper gegebenenfalls mindestens einmal calciniert, über eine Zeit von im allgemeinen 0,5 bis 10 h, bevorzugt 0,5 bis 2 Stunden. Die Temperatur bei diesem mindestens einen Calcinierschritt liegt im allgemeinen im Bereich von 200 bis 600 °C, bevorzugt im Bereich von 250 bis 500 °C und besonders bevorzugt im Bereich von 300 bis 400 °C.

Bei Einsatz als Katalysator in der oxidischen Form wird im allgemeinen der Formkörper vor Beschickung mit dem zu racemisierenden Amin I mit reduzierenden Gasen, beispielsweise Wasserstoff, vorzugsweise Wasserstoff-Inertgasgemischen, insbesondere Wasserstoff/Stickstoffgemischen, bei Temperaturen im Bereich von 100 bis 500 °C, bevorzugt im Bereich von 150 bis 350 °C und insbesondere im Bereich von 180 bis 200 °C vorreduziert (Aktivierung). Bevorzugt wird dabei ein Gemisch mit einem Wasserstoffanteil im Bereich von 1 bis 100 Vol.-%, besonders bevorzugt im Bereich von 1 bis 50 Vol.-%, verwendet.

In einer bevorzugten Ausführungsform wird der Katalysatorformkörper vor seinem Einsatz in an sich bekannter Weise durch Behandlung mit reduzierenden Medien aktiviert. Das Aktivieren erfolgt entweder vorab in einem Reduktionsofen oder nach dem Einbau im Reaktor. Ist der Katalysator vorab im Reduktionsofen aktiviert worden, wird er in den Reaktor eingebaut und direkt unter Wasserstoffdruck mit dem zu racemisierenden Amin I beschickt.

Die Reste R¹, R² und R³, wobei R¹ und R² verschieden sind, bedeuten unabhängig voneinander Alkyl-, Cycloalkyl-, Arylalkyl-, Aryl-, Heteroarylreste und heterocyclische Reste und R³ zusätzlich Wasserstoff (H), wobei die Reste unter den Reaktionsbedingungen inerte Substituenten, ausgewählt aus der Gruppe Alkyl, Cycloalkyl, Alkoxy, Aryloxy, Amino, Alkylamino und Dialkylamino, tragen können.

R¹, R² und R³ bedeuten vorzugsweise:
- linearer oder verzweigter Alkylrest, wie C₁₋₂₀-Alkyl, besonders bevorzugt C₁₋₁₂-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, Cyclopentylmethyl, n-Heptyl, iso-Heptyl, Cyclohexylmethyl, n-Octyl, 2-Ethyl-hexyl, n-Nonyl, iso-Nonyl, n-Decyl, iso-Decyl, n-Undecyl, n-Dodecyl, iso-Dodecyl,
   ganz besonders bevorzugt C₁₋₈-Alkyl, wie Methyl, Ethyl, n-Propyl, iso-Propyl, n-Butyl, iso-Butyl, sec.-Butyl, tert.-Butyl und 2-Ethyl-hexyl,
- Cycloalkylrest, bevorzugt C₃₋₈-Cycloalkyl, wie Cyclopropyl, Cyclobutyl, Cyclopentyl, Cyclohexyl, Cycloheptyl und Cyclooctyl, besonders bevorzugt Cyclopentyl, Cyclohexyl und Cyclooctyl,
   ganz besonders bevorzugt Cyclopentyl und Cyclohexyl,
- Arylalkylrest, bevorzugt C₇₋₂₀-Arylalkyl, wie Benzyl, 1-Phenethyl, 2-Phenethyl, 1-Naphthylmethyl, 2-Naphthylmethyl, Phenanthrylmethyle, 4-tert.-Butyl-phenyl-methyl, 1-Phenylpropyl, 2-Phenylpropyl, 3-Phenylpropyl, 1-Phenylbutyl, 2-Phenylbutyl, 3-Phenylbutyl und 4-Phenylbutyl,
   besonders bevorzugt Benzyl, 1-Phenethyl und 2-Phenethyl,
- aromatischer Rest, bevorzugt C₆₋₂₀-Aryl, wie Phenyl, 1-Naphthyl, 2-Naphthyl, 1-Anthryl, 2-Anthryl, 9-Anthryl, besonders bevorzugt Phenyl, 1-Naphthyl und 2-Naphthyl, besonders bevorzugt Phenyl,
- heteroaromatischer Rest, bevorzugt C₃₋₁₅-Heteroaryl, wie 2-Pyridinyl, 3-Pyridinyl, 4-Pyridinyl, Chinolinyl, Pyrazinyl, Pyrrol-3-yl, Thienyl, Imidazol-2-yl, 2-Furanyl und 3-Furanyl, und
- heterocyclischer Rest, bevorzugt C₃₋₁₅-Heterocycloalkyl, wie N-Alkyl-piperidin-3-yl, N-Alkyl-piperidin-4-yl, N,N'-Dialkylpiperazin-2-yl, Tetrahydrofuran-3-yl und N-Alkyl-pyrrolidin-3-yl,
wobei in diesen Fällen die Reste R unabhängig voneinander unter den Reaktionsbedingungen inerte Substituenten tragen können, wie z.B. C₁₋₂₀-Alkyl, C₃₋₈-Cycloalkyl, C₁₋₂₀-Alkoxy,C₆₋₂₀-Aryloxy, Amino, C₁₋₂₀-Alkylamino und C₂₋₂₀-Dialkylamino.

Dabei kann die Anzahl dieser Substituenten in R in Abhängigkeit von der Art des Restes 0 bis 5, vorzugsweise 0 bis 3, insbesondere 0, 1 oder 2 betragen. Als Substituenten kommen insbesondere in Betracht:
- C₁₋₂₀-Alkyl, wie oben definiert,
- C₃₋₈-Cycloalkyl, wie oben definiert,
- C₁₋₂₀-Alkoxy, bevorzugt C₁₋₈-Alkoxy, wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, neo-Pentoxy, 1,2-Dimethylpropoxy, n-Hexoxy, iso-Hexoxy, sec.-Hexoxy, n-Heptoxy, iso-Heptoxy, n-Octoxy, iso-Octoxy,
   besonders bevorzugt C₁₋₄-Alkoxy, wie Methoxy, Ethoxy, n-Propoxy, iso-Propoxy, n-Butoxy, iso-Butoxy, sec.-Butoxy und tert.-Butoxy,
- C₆₋₂₀-Aryloxy, wie Phenoxy, 1-Naphthoxy und 2-Naphthoxy, bevorzugt Phenoxy,
- Amino (―NH₂),
- C₁₋₂₀-Alkylamino, bevorzugt C₁₋₁₂-Alkylamino, besonders C₁₋₈-Alkylamino, wie Methylamino, Ethylamino, n-Propylamino, iso-Propylamino, n-Butylamino, iso-Butylamino, tert.-Butylamino, Cyclopentylamino, Cyclohexylamino, und
- C₂₋₂₀-Dialkylamino, bevorzugt C₂₋₁₂-Dialkylamino, besonders C₂₋₈-Dialkylamino, wie N,N-Dimethylamino, N,N-Diethylamino, N,N-Di-n-propylamino, N,N-Di-iso-propylamino, N,N-Di-n-butylamino, N-Ethyl-N-methylamino, N-Methyl-N-propylamino, Dicyclohexylamino.

R³ bedeutet ganz besonders bevorzugt Wasserstoff (H).

### Beispiele für im erfindungsgemäßen Verfahren einsetzbare Amine I sind:

1-Methoxy-2-aminopropan (MOIPA), 2-Amino-3-methylbutan, 2-Amino-3,3-dimethylbutan, 1-Phenylethylamin, 1-Naphthylethylamin, 2-Naphthylethylamin, 1-Phenylpropylamin, 2-Amino-1-phenylpropan, 2-Amino-1-(p-hydroxyphenyl)-propan, 2-Amino-1-(p-trifluormethylphenyl)-propan, 2-Amino-1-cyclohexylpropan, 2-Amino-6-methylheptan, 2-Aminoheptan, 2-Amino-4-methylhexan, 1-(4-Methylphenyl)ethylamin, 1-(4-Methoxyphenyl)ethylamin, 1-(3-Methoxyphenyl)ethylamin, 1-Aminotetralin, *trans*-1-Amino-2-benzyloxycyclopentan und *trans*-1-Amino-2-benzyloxy-cyclohexan.

Besonders bevorzugte Amine I sind 2-Butylamine und primäre β-Alkoxy-alkylamine, bevorzugt primäre β-Alkoxy-alkylamine mit R¹ = 1-Alkoxy-substituiertes Alkyl, insbesondere 1-(C₁₋₂₀-Alkoxy, wie oben definiert)-substituiertes C₁₋₂₀-Alkyl (wie oben definiert), R² = Alkyl, insbesondere C₁₋₂₀-Alkyl (wie oben definiert), und R³ = H.

### Beispiele hierfür sind 1-Methoxy-2-aminopropan (MOIPA), 2-Amino-3-methylbutan und 2-Amino-3,3-dimethylbutan.

In einer besonderen Variante wird im erfindungsgemäßen Verfahren ein optisch aktives Amin I eingesetzt, das durch Spaltung eines von diesem optisch aktiven Amin abgeleiteten Amids, das bei der Herstellung des entsprechenden Enantiomeren von I (bezogen auf das in I gezeigte asymmetrische C-Atom) durch (a) enantioselektive Acylierung des racemischen Amins I mit einem Ester, dessen Säurekomponente ein Fluor-, Stickstoff-, Phosphor-, Sauerstoffoder Schwefelatom in Nachbarschaft des Carbonylkohlenstoffatoms trägt, in Gegenwart einer Hydrolase und (b) Trennung des resultierenden Gemisches aus optisch aktivem Amin I und Amid anfällt, erhalten wurde.

In einer weiteren besonderen Variante wird im erfindungsgemäßen Verfahren ein optisch aktives Amin I eingesetzt, das bei der Herstellung des entsprechenden Enantiomeren von I (bezogen auf das in I gezeigte asymmetrische C-Atom) durch (a) enantioselektive Acylierung des racemischen Amins I mit einem Ester, dessen Säurekomponente ein Fluor-, Stickstoff-, Phosphor-, Sauerstoff- oder Schwefelatom in Nachbarschaft des Carbonylkohlenstoffatoms trägt, in Gegenwart einer Hydrolase, (b) Trennung des resultierenden Gemisches aus optisch aktivem Amin I und Amid und (c) Gewinnung des entsprechenden anderen Enantiomers von I durch Spaltung des Amids erhalten wurde.

Die Verfahren zur Herstellung von optisch aktiven Aminen I aus den entsprechenden Racematen durch (a) enantioselektive Acylierung des racemischen Amins I mit einem Ester, dessen Säurekomponente ein Fluor-, Stickstoff-, Phosphor-, Sauerstoff- oder Schwefelatom in Nachbarschaft des Carbonylkohlenstoffatoms trägt, in Gegenwart einer Hydrolase und (b) Trennung des resultierenden Gemisches aus optisch aktivem Amin I und Amid und (c) Gewinnung des entsprechenden anderen Enantiomers von I durch Spaltung des Amids sind in WO 95/08636 und WO 96/23894 beschrieben.

Bei der Hydrolase handelt es sich beispielsweise um eine Lipase, inbesondere eine mikrobielle Lipase. Bei dem Ester handelt es beispielsweise um einen C₁₋₁₂-Alkylester von C₁₋₄-Alkoxyessigsäuren, wie Methoxyessigsäureethylester.

Die Spaltung des vom optisch aktiven Amin I abgeleiteten Amids unter Erhalt der Konfiguration des Chiralitätszentrums kann durch Hydrolyse erfolgen, beispielsweise durch Hydrolyse in Gegenwart eines Polyols oder eines Aminoalkohols und eines Alkali- oder Erdalkalimetallhydroxids gemäß WO 97/10201.

Diese besonderen Verfahrensvarianten gestalten sich besonders ökonomisch, da nach der Herstellung des gewünschten Enantiomers des Amins I, z. B. gemäß WO 95/08636 oder WO 96/23894, das verbliebene, nicht gewünschte Enantiomer von I gemäß den Verfahren dieser Anmeldung racemisiert und erneut in den Verfahren zur Herstellung des gewünschten Enantiomers von I, z. B. gemäß WO 95/08636 oder WO 96/23894, eingesetzt wird. Auf diese Weise wird es möglich, insgesamt mehr als 50 % des gewünschten Enantiomers aus dem racemischen Amin I zu erhalten. (Vergleiche hierzu auch die Ausführungen auf Seite 1 der Beschreibung, 2. Absatz).

### Beispiele

### Beispiel 1: Herstellung des Katalysators

### 1a) Herstellung des Trägers

Zu 649 g einer gut gerührten wässrigen Lösung von Zinknitrat mit einem Zink-Gehalt von 14,5 Gew.-% wurden 450 g Al(NO₃)₃ * 9 H₂O zugegeben und das Gemisch mit Wasser auf ein Volumen von 1,25 l gebracht, um das Aluminiumsalz in Lösung zu bringen (Lösung A). In einem separaten Gefäß wurden 474 g wasserfreies Soda in Wasser gelöst und die Lösung auf 2 l mit Wasser aufgefüllt (Lösung B).

Lösung A und Lösung B wurden auf 50 °C erhitzt und über getrennte Leitungen in ein Fällgefäß, das eine auf 50 °C erhitzte, gut gerührte Lösung von 20 g NaHCO₃ in 350 ml Wasser enthielt, geleitet. Hierbei wurde durch entsprechende Einstellung der Zufuhrgeschwindigkeiten der Lösungen A und B der pH-Wert innerhalb von ca. 3 Minuten auf 6,8 gebracht. Unter Konstanthaltung des pH-Wertes bei 6,8 und der Temperatur bei 50 °C wurde die gesamte Lösung A mit Soda zur Reaktion gebracht. Die so gebildete Suspension wurde anschließend 3 Stunden lang nachgerührt, wobei der pH-Wert durch gelegentliche Zugabe von verdünnter Salpetersäure bei 6,8 gehalten wurde. Die Suspension wurde filtriert und mit destilliertem Wasser so lange gewaschen, bis der Nitratgehalt des Waschwassers kleiner 10 ppm betrug. Der Filterkuchen wurde 16 h lang bei 120 °C getrocknet und anschließend 1 h lang bei 425 °C calciniert.

### 1b) Herstellung des Katalysators

Ein Gemisch aus 432 g einer salpetersauren Kupfernitratlösung mit einem Kupfergehalt von 15,5 Gew.-% und 95 g einer salpetersauren Zinknitratlösung mit einem Zink-Gehalt von 14,5 Gew.-% wurde mit Wasser auf 500 ml verdünnt und auf 70 °C erwärmt. Unter Rühren wurden 25,1 g des oben beschriebenen pulverförmigen calcinierten Trägers während ca. 5 Minuten langsam zugegeben und die so erhaltene milchige Suspension 15 Minuten lang gerührt (Suspension C).

In einem separaten Gefäß wurden 474 g wasserfreies Soda in Wasser gelöst und die Lösung auf 2 l mit Wasser aufgefüllt und auf 70 °C erhitzt (Lösung D). Suspension C und Lösung D wurden über getrennte Leitungen in ein Fällgefäß, das mit einem Rührer versehen war und 350 ml auf 70 °C erhitztes Wasser enthielt, geleitet. Hierbei wurde durch entsprechende Einstellung der Zufuhrgeschwindigkeiten der Suspension C und Lösung D der pH-Wert auf 7,4 gebracht.

Unter Konstanthaltung des pH-Wertes bei 7,4 und der Temperatur bei 70 °C wurde die gesamte Suspension C mit Soda zur Reaktion gebracht. Die so gebildete Suspension wurde anschließend 2 Stunden lang nachgerührt, wobei der pH-Wert durch gelegentliche Zugabe von verdünnter Salpetersäure bzw. Sodalösung D bei 7,4 gehalten wurde. Die Suspension wurde filtriert und mit destilliertem Wasser so lange gewaschen, bis der Nitratgehalt des Waschwassers kleiner 10 ppm betrug.

Der Filterkuchen wurde 16 h lang bei 120 °C getrocknet und anschließend 1 h lang bei 430 °C calciniert. Das so erhaltene braunschwarze Katalysatorpulver wurde mit 1,5 Gew.-% Graphit und 5 Gew.-% Kupferpulver (Typ FFL Nr. 10914 der Norddeutschen Affinerie) mit einer BET-Oberfläche von 0,23 m2/g und einer Partikelgrößenverteilung, bei der 92 % der Partikel im Größenbereich 10 bis 100 µm liegen) gemischt und zu Tabletten von 3 mm Durchmesser und 3 mm Höhe verpresst. Die Tabletten wurden schließlich 1 h lang bei 330 °C calciniert.

Der so hergestellte Katalysator hatte die chemische Zusammensetzung 66 % CuO, 24 % ZnO, 5 % Al₂O₃ und 5 % Cu (Angaben in Gew.-%).

### Beispiel 2: kontinuierliche Racemisierung von (S)-MOIPA

In einem mit Öl beheizten Rohrreaktor (40 x 3500 mm) wurden 1000 ml (Schüttvolumen) des Katalysators aus Beispiel 1 auf eine Schicht von 250 ml V2A-Ringen eingefüllt und anschließend mit 3000 ml V2A-Ringen bedeckt. Der Katalysator wurde zunächst mit einem Gemisch von Wasserstoff und Stickstoff (5 : 95 Vol.-%) und anschließend mit reinem Wasserstoff bei Temperaturen von 180-220 °C aktiviert. Bei 230 °C und 16 bar (absolut) Wasserstoff wurden 200 ml (S)-MOIPA (Reinheit nach GC: 95,4 % (S)-MOIPA) pro Stunde und so viel Ammoniak durch den Reaktor eingeleitet, dass der Ammoniak-Anteil im Kreisgas ca. bei 20 Vol.-% lag. Das Frischgas Wasserstoff und das Kreisgas betrugen hierbei jeweils 300 Nl/h und 6,4 Nm³/h (Nl = Normliter, Nm³ = Normkubikmeter: auf Normalbedingungen umgerechnetes Volumen). Nachdem der stationäre Zustand erreicht wurde, wurde eine Probe aus den Reaktionsausträgen genommen und gaschromatographisch analysiert. Hieraus ergaben sich eine Racemisierungsausbeute von 94 % und ein Racemisierungsgrad von 96,2 % (51,9 % (S)- und 48,1% (R)-MOIPA).

Die in den Beispielen angewandten GC-Bedingungen zur Analyse des Reaktoraustrags waren: 30 m RTX-5 Amine 0,32 mm, 1,5 µm, 80 °C/4 Min. - 10 Min. - 280 °C/5 Min.

Die in den Beispielen verwendete GC-Methode zur Bestimmung des Enantiomerenüberschusses von MOIPA war wie folgt:

MOIPA wird im analytischen Maßstab mit Acetanhydrid derivatisiert. Das Rohprodukt wird per GC analysiert.
- GC-Bedingungen:: Gerät: HP-5890-II
Säule: 20 m Chiraldex G-TA (Fa. Astec)
Trägergas: Helium
Vordruck: 65 kPa
Injektortemperatur: 250 °C
Detektor: FID
- Detektortemperatur:: 275 °C
- Injizierte Menge:: 1 µl
- Temperaturprogramm:: 125 °C isotherm
- Retentionszeiten:: MOIPA (Acetamid):(S)-Enantiomer: 4,5 Min.,
- (R)-Enantiomer:: 5,3 Min.

### Beispiele 3-8: kontinuierliche Racemisierung von (S)-MOIPA

In einer kontinuierlich betriebenen Laborapparatur (Rohrreaktor 60 ml) ohne Rückführung wurde einer der Katalysatoren 1-5 (siehe unten) in reduzierter und passivierter Form eingebaut und 6 h bei 200 °C unter Wasserstoffatmosphäre reduziert (Normaldruck). Dann wurde bei 16 bar Wasserstoffdruck angefahren (18 Nl/h Wasserstoff). Nach Einstellung der o.g. Reaktionsbedingungen wurde Ammoniak angefahren (bei Belastung 0,2 kg/l*h: 17 g Ammoniak, bei Belastung 0,4 kg/l*h: 34 g Ammoniak, bei Belastung 0,46 kg/l*h: 38 g Ammoniak). Der Austrag wurde in einen Abscheider entspannt und gaschromatographisch analysiert.

Folgende Katalysatoren wurden eingesetzt (Angaben in Gew.-%) :
Katalysator 1: 53 % CuO, 5 % Cu, 42 % TiO₂, gemäß DE-A-198 59 776
Katalysator 2: 53 % CuO, 47 % Al₂O₃, gemäß EP-A-691 157, Seite 5, Zeile 55, bis Seite 6, Zeile 11
Katalysator 3: 70 % CuO, 24,5 % ZnO, 5,5 % Al₂O₃, gemäß Beispiel aus US-A-3,923,694
Katalysator 4: 45 % CuO, 10 % NiO, 45 % Al₂O₃, gemäß EP-A-514 692
Katalysator 5: 66 % CuO, 24 % ZnO, 5 % Al₂O₃, 5 % Cu-Pulver, gemäß Beispiel 1

### Ergebnisse:

| | | | | | GC-Analysen | | |
|---|---|---|---|---|---|---|---|
| Beisp. Nr. | Katalysator | Belast. in kg/l*h | Temp. in °C | ee-Wert in % | MOIPA RT=4,7 | RT=5,9 | Dimer RT=12,6 |
| 3 | 1 | 0,46 | 200 | 88,6 | 98,2 | 0,2 | 0,9 |
| 4 | 2 | 0,2 | 200 | 83 | 97,7 | 0,3 | 1 |
| 5 | 3 | 0,2 | 200 | 0 | 84,35 | 2,01 | 12,59 |
| 6 | 4 | 0,2 | 200 | 0 | 68,9 | 1,87 | 26,67 |
| 7 | 5 | 0,2 | 220 | 0,2 | 80,68 | 2,36 | 10,12 |
| 8 | 5 | 0,4 | 220 | 1,2 | 89,24 | 1,32 | 8,44 |
| [kg/l*h = kg MOIPA im Reaktorfeed pro Liter Katalysator (Schüttvolumen) und pro Stunde | | | | | | | |
| RT = Retentionszeit im GC in Min. | | | | | | | |
| Die Angaben zur Zusammensetzung des Reaktionsaustrags (GC-Analysen) erfolgen in GC-Fl.% | | | | | | | |
| Dimer = Diamin (sekundäres Amin)] | | | | | | | |

Bei den Beispielen Nr. 3, 4 und 6 (Katalysatoren 1, 2 und 4) handelt es sich um Vergleichsbeispiele.

Die Beispiele zeigen, dass für (S)-MOIPA unter den Reaktionsbedingungen mit den Cu-Katalysatoren 1 und 2 verbesserungswürdige Racemisierungsgrade (ee-Werte: 83 bzw. 88,6 %) erzielt wurden.

Unter Verwendung des nickelhaltigen Cu-Katalysators 4 wurde zwar eine vollständige Racemisierung (ee-Wert: 0 %) erreicht, die Racemisierungsausbeute (Racematausbeute) war jedoch verbesserungswürdig (nur 68,9 GC-Fl.% MOIPA und hoher Dimer-Anteil von 26,67 GC-Fl.% im Austrag).

Die erfindungsgemäßen Cu/Zn-Katalysatoren 3 und 5 lieferten bei hoher Raum-Zeit-Ausbeute sowohl einen hohen Racemisierungsgrad als auch eine hohe Racemisierungsausbeute.

### Beispiel 9 (kombinierte Fahrweise): kontinuierliche Racemisierung von (S)-MOIPA und in situ Aminierung von 1-Methoxy-2-propanol

In einem mit Öl beheizten Rohrreaktor (48 x 2100 mm) wurden 1000 ml des Katalysators aus Beispiel 1 auf eine Schicht von 60 ml V2A-Ringen eingefüllt und anschließend mit 1330 ml V2A-Ringen bedeckt. Der Katalysator wurde zunächst mit einem Gemisch von Wasserstoff und Stickstoff und schließlich mit reinem Wasserstoff bei Temperaturen von 180-220 °C aktiviert. Bei 230 °C und 16 bar (abs.) Wasserstoff wurden pro Stunde 500 ml eines Gemisches von 1-Methoxy-2-propanol und (S)-MOIPA im Gewichtverhältnis von 4:1 zudosiert und so viel Ammoniak durch den Reaktor eingeleitet, dass der Ammoniak-Anteil im Kreisgas ca. bei 40 Vol.-% lag. Das Frischgas Wasserstoff und das Kreisgas betrugen hierbei jeweils 300 Nl/h und 6,4 Nm³/h. Nachdem der stationäre Zustand erreicht wurde, wurde eine Probe aus den Reaktionsausträgen genommen und gaschromatographisch analysiert. Hieraus ergaben sich eine (R,S)-MOIPA-Selektivität von 91 % und ein Racemisierungsgrad von 99,8 % [49,9 % (R)-MOIPA, 50,1% (S)-MOIPA] bei einem Umsatz von 99 %.

### Beispiel 10 (Vergleichsbeispiel):

### Versuch zur Racemisierung von (R)-MOIPA analog DE-A-29 03 589

In einem 0,3 l Autoklaven wurden 10 g (R)-MOIPA (112 mmol), 70 ml Tetrahydrofuran und 1 g Raney-Cobalt vorgelegt. Mit Wasserstoff wurde ein Druck von 20 bar eingestellt. Der Autoklav wurde auf 160 °C aufgeheizt und der H₂-Druck auf 50 bar erhöht. Nach 12 h bei diesen Bedingungen wurde auf Raumtemperatur abgekühlt, der Katalysator abfiltriert und das Tetrahydrofuran am Rotationsverdampfer abgezogen. Die Rückstandsmenge betrug 2,5 g.

Bestimmung des Enantiomerenüberschusses (ee) durch HPLC-Analyse: 4,8 %
(S)-MOIPA = 47,6 Fl.%
(R)-MOIPA = 52,4 Fl.%

| GC-Analyse [F1.%]: | |
|---|---|
| Tetrahydrofuran | 0,2 |
| Methoxyisopropanol | 2,2 |
| (R)- + (S)-MOIPA | 68,3 |
| Octylamin | 3,7 |
| Octanol | 10,0 |
| Summe unbek. Verb. | 15,6 |
| Racemisierungsgrad | 90 % |
| Racematausbeute | 61 % |

### Beispiel 11 (Vergleichsbeispiel):

### Versuch zur Racemisierung von (S)-3,3-Dimethyl-2-amino-butan (Pinacolylamin) analog DE-A-29 03 589

10 g (S)-Pinacolylamin (99 % ee) wurden zusammen mit 60 g THF und 1 g Raney-Cobalt in einem 0,3 l Rohrautoklaven vermischt und unter Wasserstoffatmosphäre bei einem Druck von 50 bar und einer Temperatur von 165 °C 12 h gerührt.

Dann wurde der Reaktorinhalt auf Raumtemperatur abgekühlt vom Katalysator abgetrennt und das Enantiomerenverhältnis über eine chirale HPLC-Säule bestimmt.

Enantiomerenüberschuss: 99 %

| GC-Analyse des Austrags (ammoniak- und wasserfrei) in GC-Fl.%: | |
|---|---|
| Pinacolon | 0,1 |
| Pinacolylamin | 99,2 |
| Pinacolol | 0,5 |
| Sonstige | 0,2 |
| Racemisierungsgrad: | 0 % |
| Racematausbeute: | 99,2 % |

### Beispiel 12: Aminierung von 1-Methoxy-2-propanol

In einem mit Öl beheizten Rohrreaktor (48 x 2100 mm) wurden 1000 ml des Katalysators aus Beispiel 1 auf eine Schicht von 60 ml V2A-Ringen eingefüllt und anschließend mit 1330 ml V2A-Ringen bedeckt. Der Katalysator wurde mit einem Gemisch von Wasserstoff und Stickstoff bzw. mit reinem Wasserstoff bei Temperaturen von 180-220 °C aktiviert. Bei 220 °C und 16 bar (abs.) Wasserstoff wurden pro Stunde 200 ml 1-Methoxy-2-propanol und so viel Ammoniak durch den Reaktor eingeleitet, dass der Ammoniak-Anteil im Kreisgas ca. bei 40 Vol.-% lag. Das Frischgas Wasserstoff und das Kreisgas betrugen hierbei jeweils 300 Nl/h und 6,4 Nm³/h. Nachdem der stationäre Zustand erreicht wurde, wurde eine Probe aus den Reaktionsausträgen genommen und gaschromatographisch analysiert. Hieraus ergab sich eine (R,S)-MOIPA-Selektivität von 94,6 % bei einem Umsatz von 99,4 %.

## Patentansprüche

1. Verfahren zur Racemisierung von optisch aktiven Aminen der Formel I 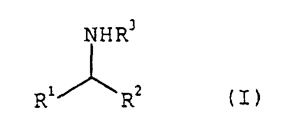 in der R¹ und R² verschieden sind und R¹, R², R³ Alkyl-, Cycloalkyl-, Arylalkyl-, Aryl-, Heteroarylreste und heterocyclische Reste bedeuten und R³ zusätzlich Wasserstoff (H) bedeuten kann, wobei die Reste Substituenten, ausgewählt aus der Gruppe Alkyl, Cycloalkyl, Alkoxy, Aryloxy, Amino, Alkylamino und Dialkylamino, tragen können, durch Umsetzung des optisch aktiven Amins I in Gegenwart von Wasserstoff und einem Hydrier- oder Dehydrierkatalysator bei erhöhter Temperatur, **dadurch gekennzeichnet, dass** der Katalysator die Aktivkomponenten Kupfer und Zinkoxid und ein Trägermaterial enthält.

2. Verfahren nach Anspruch 1, **dadurch gekennzeichnet, dass** der Katalysator ein oxidisches Trägermaterial, ausgewählt aus der Gruppe Aluminiumoxid, Siliziumdioxid, Titandioxid, Zirkoniumdioxid, Magnesiumoxid, Eisenoxid und Cerdioxid, oder Kohlenstoff oder Gemische hiervon, enthält.

3. Verfahren nach den Ansprüchen 1 oder 2, **dadurch gekennzeichnet, dass** der Katalysator Zink-Aluminium-Spinell enthält.

4. Verfahren nach einem der Ansprüche 1 bis 3, **dadurch gekennzeichnet, dass** der Katalysator nach der Trocknung und vor der Aktivierung mit einem Reduktionsmittel
20 bis 90 Gew.-% sauerstoffhaltige Verbindungen des Kupfers, berechnet als CuO,
9 bis 60 Gew.-% sauerstoffhaltige Verbindungen des Zinks, berechnet als ZnO, und
1 bis 60 Gew.-% Aluminiumoxid, berechnet als Al₂O₃,
jeweils bezogen auf das Gesamtgewicht aller oxidischen Bestandteile des Katalysators,
enthält.

5. Verfahren nach einem der Ansprüche 1 bis 3, **dadurch gekennzeichnet, dass** der Katalysator nach der Trocknung und vor der Aktivierung mit einem Reduktionsmittel
40 bis 85 Gew.-% sauerstoffhaltige Verbindungen des Kupfers, berechnet als CuO,
13 bis 40 Gew.-% sauerstoffhaltige Verbindungen des Zinks, berechnet als ZnO, und
2 bis 35 Gew.-% Aluminiumoxid, berechnet als Al₂O₃,
jeweils bezogen auf das Gesamtgewicht aller oxidischen Bestandteile des Katalysators,
enthält.

6. Verfahren nach einem der Ansprüche 1 bis 5, **dadurch gekennzeichnet, dass** der Katalysator nach der Trocknung und vor der Aktivierung mit einem Reduktionsmittel die Bestandteile sauerstoffhaltige Verbindungen des Kupfers, sauerstoffhaltige Verbindungen des Zinks und Aluminiumoxid zusammen in einer Menge von mindestens 80 Gew.-%, bezogen auf das Gesamtgewicht aller oxidischen Bestandteile des Katalysators, enthält.

7. Verfahren nach einem der Ansprüche 1 bis 6, **dadurch gekennzeichnet, dass** man die Umsetzung bei Temperaturen von 100 bis 300 °C durchführt.

8. Verfahren nach einem der Ansprüche 1 bis 7, **dadurch gekennzeichnet, dass** man die Umsetzung bei Drücken von 0,1 bis 30 MPa durchführt.

9. Verfahren nach einem der Ansprüche 1 bis 8, **dadurch gekennzeichnet, dass** man die Umsetzung in Gegenwart des Amins der Formel R³NH₂, bei dem der Rest R³ dem Rest R³ des optisch aktiven Amins I entspricht, durchführt.

10. Verfahren nach Anspruch 9, **dadurch gekennzeichnet, dass** man gleichzeitig in situ den sekundären Alkohol der Formel II und/oder das unsymmetrische Keton der Formel III 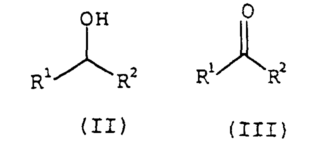 bei denen die Reste R¹ und R² den Resten R¹ und R² des Amins I entsprechen, zum racemischen Amin I umsetzt.

11. Verfahren nach einem der Ansprüche 1 bis 10, **dadurch gekennzeichnet, dass** man als optisch aktives Amin I ein primäres β-Alkoxy-alkylamin mit R¹ = 1-Alkoxy-substituiertes Alkyl, R² = Alkyl und R³ = H einsetzt.

12. Verfahren nach Anspruch 11, **dadurch gekennzeichnet, dass** man als optisch aktives Amin I 1-Methoxy-2-propylamin einsetzt.

13. Verfahren nach einem der Ansprüche 1 bis 10, **dadurch gekennzeichnet, dass** man als optisch aktives Amin I 3-Methyl-2-butylamin oder 3,3-Dimethyl-2-butylamin einsetzt.

14. Verfahren nach Anspruch 10, **dadurch gekennzeichnet, dass** man als optisch aktives Amin I 1-Methoxy-2-propylamin und als sekundären Alkohol II 1-Methoxy-2-propanol einsetzt.

15. Verfahren nach einem der Ansprüche 1 bis 14, **dadurch gekennzeichnet, dass** das optisch aktive Amin I durch Spaltung eines von diesem optisch aktiven Amin abgeleiteten Amids, das bei der Herstellung des entsprechenden Enantiomeren von I durch (a) enantioselektive Acylierung des racemischen Amins I mit einem Ester, dessen Säurekomponente ein Fluor-, Stickstoff-, Phosphor-, Sauerstoff- oder Schwefelatom in Nachbarschaft des Carbonylkohlenstoffatoms trägt, in Gegenwart einer Hydrolase und (b) Trennung des resultierenden Gemisches aus optisch aktivem Amin I und Amid anfällt, erhalten wurde.

16. Verfahren nach einem der Ansprüche 1 bis 15, **dadurch gekennzeichnet, dass** das optisch aktive Amin I bei der Herstellung des entsprechenden Enantiomeren von I durch (a) enantioselektive Acylierung des racemischen Amins I mit einem Ester, dessen Säurekomponente ein Fluor-, Stickstoff-, Phosphor-, Sauerstoff- oder Schwefelatom in Nachbarschaft des Carbonylkohlenstoffatoms trägt, in Gegenwart einer Hydrolase, (b) Trennung des resultierenden Gemisches aus optisch aktivem Amin I und Amid und (c) Gewinnung des entsprechenden anderen Enantiomers von I durch Spaltung des Amids erhalten wurde.

## Claims

1. A process for racemizing optically active amines of the formula I 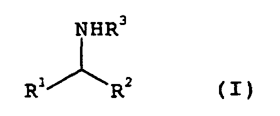 where R¹ and R² are different and R¹, R², R³ are alkyl, cycloalkyl, arylalkyl, aryl, heteroaryl and heterocyclic radicals and R³ may also be hydrogen (H), with the radicals being able to bear substituents selected from the group consisting of alkyl, cycloalkyl, alkoxy, aryloxy, amino, alkylamino and dialkylamino, by reacting the optically active amine I in the presence of hydrogen and a hydrogenation or dehydrogenation catalyst at elevated temperature, wherein the catalyst comprises the active components copper and zinc oxide and a support material.

2. A process as claimed in claim 1, wherein the catalyst comprises an oxidic support material selected from the group consisting of aluminum oxide, silicon dioxide, titanium dioxide, zirconium dioxide, magnesium oxide, iron oxide and cerium dioxide, or carbon or a mixture thereof.

3. A process as claimed in claim 1 or 2, wherein the catalyst comprises zinc-aluminum spinel.

4. A process as claimed in any of claims 1 to 3, wherein the catalyst after drying and before activation with a reducing agent comprises
from 20 to 90% by weight of oxygen-containing compounds of copper, calculated as CuO,
from 9 to 60% by weight of oxygen-containing compounds of zinc, calculated as ZnO, and
from 1 to 60% by weight of aluminum oxide, calculated as Al₂O₃,
in each case based on the total weight of all oxidic constituents of the catalyst.

5. A process as claimed in any of claims 1 to 3, wherein the catalyst after drying and before activation with a reducing agent comprises
from 40 to 85% by weight of oxygen-containing compounds of copper, calculated as CuO,
from 13 to 40% by weight of oxygen-containing compounds of zinc, calculated as ZnO, and
from 2 to 35% by weight of aluminum oxide, calculated as Al₂O₃,
in each case based on the total weight of all oxidic constituents of the catalyst.

6. A process as claimed in any of claims 1 to 5, wherein the catalyst after drying and before activation with a reducing agent comprises the constituents oxygen-containing compounds of copper, oxygen-containing compounds of zinc and aluminum oxide in a combined amount of at least 80% by weight, based on the total weight of all oxidic constituents of the catalyst.

7. A process as claimed in any of claims 1 to 6, wherein the reaction is carried out at from 100 to 300°C.

8. A process as claimed in any of claims 1 to 7, wherein the reaction is carried out at pressures of from 0.1 to 30 MPa.

9. A process as claimed in any of claims 1 to 8, wherein the reaction is carried out in the presence of the amine of the formula R³NH₂ in which the radical R³ corresponds to the radical R³ of the optically active amine I.

10. A process as claimed in claim 9, wherein the secondary alcohol of the formula II and/or the unsymmetrical ketone of the formula III 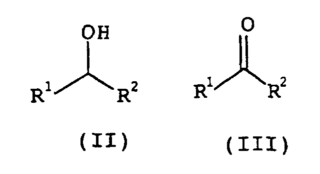 in which the radicals R¹ and R² correspond to the radicals R¹ and R² of the amine I, are/is reacted simultaneously in situ to form the racemic amine I.

11. A process as claimed in any of claims 1 to 10, wherein the optically active amine I used is a primary β-alkoxyalkylamine in which R¹ = 1-alkoxy-substituted alkyl, R² = alkyl and R³ = H.

12. A process as claimed in claim 11, wherein the optically active amine I used is 1-methoxy-2-propylamine.

13. A process as claimed in any of claims 1 to 10, wherein the optically active amine I used is 3-methyl-2-butylamine or 3,3-dimethyl-2-butylamine.

14. A process as claimed in claim 10, wherein 1-methoxy-2-propylamine is used as optically active amine I and 1-methoxy-2-propanol is used as secondary alcohol II.

15. A process as claimed in any of claims 1 to 14, wherein the optically active amine I has been obtained by cleavage of an amide which is derived from this optically active amine and is obtained in the preparation of a particular enantiomer of I by (a) enantioselective acylation of the racemic amine I by means of an ester whose acid component bears a fluorine, nitrogen, phosphorus, oxygen or sulfur atom in a vicinal position relative to the carbonyl carbon in the presence of a hydrolase and (b) separation of the resulting mixture of optically active amine I and amide.

16. A process as claimed in any of claims 1 to 15, wherein the optically active amine I has been obtained in the preparation of a particular enantiomer of I by (a) enantioselective acylation of the racemic amine I by means of an ester whose acid component bears a fluorine, nitrogen, phosphorus, oxygen or sulfur atom in a vicinal position relative to the carbonyl carbon in the presence of a hydrolase, (b) separation of the resulting mixture of optically active amine I and amide and (c) isolation of the other enantiomer of I by cleavage of the amide.

## Revendications

1. Procédé de racémisation d'amines optiquement actives de la formule I : 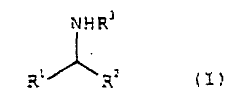 dans laquelle R¹ et R² sont différents et R¹, R² et R³ représentent des radicaux alkyle, cycloalkyle, arylalkyle, aryle, hétéroaryle et hétérocycliques, R³ pouvant représenter en outre de l'hydrogène (H), les radicaux pouvant porter des substituants choisis parmi le groupe des radicaux alkyle, cycloalkyle, alcoxy, aryloxy, amino, alkylamino et dialkylamino, par transformation de l'amine optiquement active I en présence d'hydrogène et d'un catalyseur d'hydrogénation ou de déshydrogénation, à température élevée, **caractérisé en ce que** le catalyseur contient les composants actifs cuivre et oxyde de zinc et une matière de support.

2. Procédé suivant la revendication 1, **caractérisé en ce que** le catalyseur contient une matière de support oxydée, choisie parmi le groupe de l'oxyde d'aluminium, du dioxyde de silicium, du dioxyde de titane, du dioxyde de zirconium, de l'oxyde de magnésium, de l'oxyde de fer et du dioxyde de cérium, ou du carbone ou leurs mélanges.

3. Procédé suivant l'une des revendications 1 et 2, **caractérisé en ce que** le catalyseur contient un spinelle de zinc-aluminium.

4. Procédé suivant l'une des revendications 1 à 3, **caractérisé en ce que** le catalyseur contient, après le séchage et avant l'activation par un réducteur,
20 à 90% en poids de composés du cuivre contenant de l'oxygène, calculés sous la forme de CuO,
9 à 60% en poids de composés du zinc contenant de l'oxygène, calculés sous la forme de ZnO, et
1 à 60% en poids d'oxyde d'aluminium, calculé sous la forme de Al₂O₃,
chaque fois par rapport au poids total de tous les composants oxydés du catalyseur.

5. Procédé suivant l'une des revendications 1 à 3, **caractérisé en ce que** le catalyseur contient, après le séchage et avant l'activation par un réducteur,
40 à 85% en poids de composés du cuivre contenant de l'oxygène, calculés sous la forme de CuO,
13 à 40% en poids de composés du zinc contenant de l'oxygène, calculés sous la forme de ZnO, et
2 à 35% en poids d'oxyde d'aluminium, calculé sous la forme de Al₂O₃,
chaque fois par rapport au poids total de tous les composants oxydés du catalyseur.

6. Procédé suivant l'une des revendications 1 à 5, **caractérisé en ce que** le catalyseur contient, après le séchage et avant l'activation par un réducteur, les composants constitués des composés du cuivre contenant de l'oxygène, des composés du zinc contenant de l'oxygène et de l'oxyde d'aluminium conjointement en une quantité d'au moins 80% en poids, par rapport au poids total de tous les composants oxydés du catalyseur.

7. Procédé suivant l'une des revendications 1 à 6, **caractérisé en ce qu'**on effectue la transformation à des températures de 100 à 300°C.

8. Procédé suivant l'une des revendications 1 à 7, **caractérisé en ce qu'**on effectue la transformation à des pressions de 0,1 à 30 MPa.

9. Procédé suivant l'une des revendications 1 à 8, **caractérisé en ce qu'**on effectue la transformation en présence de l'amine de la formule R³NH₂, dans laquelle le radical R³ correspond au radical R³ de l'amine optiquement active I.

10. Procédé suivant la revendication 9, **caractérisé en ce qu'**on fait réagir simultanément in situ l'alcool secondaire de la formule II et/ou la cétone dissymétrique de la formule III : 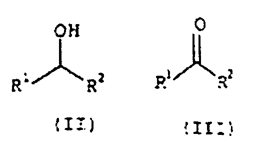 dans lesquelles les radicaux R¹ et R² correspondent aux radicaux R¹ et R² de l'amine I, pour former l'amine racémique I.

11. Procédé suivant l'une des revendications 1 à 10, **caractérisé en ce que**, comme amine optiquement active I, on met en oeuvre une β-alcoxy-alkylamine primaire où R¹ = alkyle substitué par un groupe 1-alcoxy, R² = alkyle et R³ = H.

12. Procédé suivant la revendication 11, **caractérisé en ce que**, comme amine optiquement active I, on met en oeuvre de la 1-méthoxy-2-propylamine.

13. Procédé suivant l'une des revendications 1 à 10, **caractérisé en ce que**, comme amine optiquement active I, on met en oeuvre de la 3-méthyl-2-butylamine ou de la 3,3-diméthyl-2-butylamine.

14. Procédé suivant la revendication 10, **caractérisé en ce que**, comme amine optiquement active I, on met en oeuvre de la 1-méthoxy-2-propylamine et, comme alcool secondaire II, du 1-méthoxy-2-propanol.

15. Procédé suivant l'une des revendications 1 à 14, **caractérisé en ce que** l'amine optiquement active I est obtenue par dissociation d'un amide dérivé de cette amine optiquement active, qui est formé au cours de la préparation de l'énantiomère correspondant de I par (a) acylation énantiosélective de l'amine racémique I avec un ester, dont la composante acide porte un atome de fluor, d'azote, de phosphore, d'oxygène ou de soufre au voisinage de l'atome de carbone du carbonyle, en présence d'une hydrolase, et (b) séparation du mélange résultant d'amine optiquement active I et d'amide.

16. Procédé suivant l'une des revendications 1 à 15, **caractérisé en ce que** l'amine optiquement active I est obtenue au cours de la préparation de l'énantiomère correspondant de I par (a) acylation énantiosélective de l'amine racémique I avec un ester, dont la composante acide porte un atome de fluor, d'azote, de phosphore, d'oxygène ou de soufre au voisinage de l'atome de carbone du carbonyle, en présence d'une hydrolase, (b) séparation du mélange résultant d'amine optiquement active I et d'amide et (c) obtention de l'autre énantiomère correspondant de I par dissociation de l'amide.
